# EUROPEAN PATENT SPECIFICATION

(11) **EP 3 989 904 B1**
(45) Date of publication and mention of the grant of the patent: **09.10.2024**
(21) Application number: 20742487.0
(22) Date of filing: 25.06.2020
(51) Int. Cl.: A61H 1/02

(54) **APPARATUS CAPABLE OF ACTUATING A DISTAL JOINT AND TRANSFERRING THE CONSTRAINING REACTIONS IN AN UNDERACTUATED SHOULDER EXOSKELETON**
VORRICHTUNG ZUR BETÄTIGUNG EINER DISTALEN VERBINDUNG UND ZUR ÜBERTRAGUNG DER EINSCHRÄNKUNGSREAKTIONEN IN EINEM UNTERBETÄTIGTEN SCHULTEREXOSKELETT
APPAREIL POUVANT ACTIONNER UNE ARTICULATION DISTALE ET TRANSFÉRER DES RÉACTIONS DE CONTRAINTE À UN EXOSQUELETTE D'ÉPAULE SOUS-ACTIONNÉ

(30) Priority: 25.06.2019 IT 201900010026
(43) Date of publication of application: 04.05.2022
(73) Proprietor: Scuola Superiore di Studi Universitari e di Perfezionamento Sant'Anna, 56127 Pisa (IT); Universität Heidelberg, 69117 Heidelberg (DE)
(72) Inventor: MASIA, Lorenzo, 69117 Heidelberg (DE); FRISOLI, Antonio, 56127 Pisa (IT); TISENI, Luca, 56127 Pisa (IT); CHIARADIA, Domenico, 56127 Pisa (IT); XILOYANNIS, Michele, 56127 Pisa (IT); SOLAZZI, Massimiliano, 56127 Pisa (IT)
(74) Representative: Leihkauf, Steffen Falk
(86) International application number: PCT/IB2020/056009
(87) International publication number: WO 2020/261167

(56) References cited:
- WO-A1-2017/093298
- WO-A1-2017/216663
- WO-A2-2013/186705
- WO-A2-2015/099858
- GB-A- 2 567 999

## Description

### TECHNICAL FIELD

The present invention relates to the technical field of upper limb exoskeletons for strengthening or for assistance in patients with motor disabilities of the upper limb, in particular to a system adapted to transmit a flexion-extension torque on the shoulder joint.

### BACKGROUND ART

The present invention finds application in the field of rehabilitation medicine and human enhancement in the industrial field.

In fact, in the industrial field, in order to reduce and prevent the onset of pathologies of the musculoskeletal system caused by an overload of work duties requiring repetitive tasks with the transport of loads and to reduce muscle fatigue in repetitive tasks, wearable support apparatuses of the exoskeletal type, either of the passive or robotic type, have spread, being capable of transferring the forces required to perform tasks from distal districts of the upper limb to districts of the body, such as the torso or legs, bypassing the transfer of the load onto the lumbar areas.

In the medical rehabilitation field, exoskeletons are instead commonly used for robot-assisted rehabilitation.

In the field of rehabilitation robots and cooperative robots for upper limbs, there is a macro-division between two types of robots, End effector based Robots (EEB Robot) and Exoskeletons.

In EEB robots, the interaction between the robot and the human body occurs only through the end effector, which is in contact with the patient's hand or forearm. The arm can be spatially positioned and/or oriented depending on the degrees of freedom available for the mechanism. Usually, the rotation axes of the robotic system joints do not correspond to those of the human body joints: this explains why EEB robots are easier to design and produce than exoskeletons are. They are also more easily adjustable to different lengths of the patient's arm.

These advantages are contrasted by the inability to uniquely determine the posture of the arm and/or the torques at the human joints knowing the robot posture: in fact, the interaction between robot and human occurs through only one point of contact.

Then, a real transition from EEBs to exoskeletons occurred. Upper limb exoskeletons are mechanisms with kinematics isomorphous to those of the arm, with the axes of the mechanical joints aligned with those of the human body joints.

They are therefore substantially a mechanical structure parallel to the arm. This feature allows exoskeletal devices to have multiple connection points (interfaces) between human and robot: the greater number of interfaces allows to accurately determine the posture of the human limb based on the posture of the robotic one. The same applies to the torques at the human joints, which can be determined and monitored individually, one by one. By means of mechanical stops at the joints, unwanted phenomena such as elbow hyperextension can be avoided. It is often possible to cover a large part of the working space reachable by the human arm with an exoskeleton, simply because the exoskeleton has kinematics similar to those of the upper limb: however, this involves the need to adjust the link lengths to the arm segment lengths.

The most important aspects to consider when designing an exoskeleton for the upper limb include the following:
The shoulder center of rotation is not fixed: the shoulder center of rotation moves during flexion and extension movements and abduction and adduction movements. If the exoskeleton is also attached to the body on the forearm, the movement of the center of rotation must be considered in order to eliminate errors due to the misalignment between the robotic joint axes and the shoulder axes.

The singular configuration of the robotic shoulder joint should not be inside the robot workspace, or at most should be at the borders thereof.

Centering the robotic shoulder joint axes on the human shoulder center of rotation, which is inside the body, is an operation which in any case cannot be carried out with accuracy and precision, and which must be repeated for each patient.

The inertia of the moving parts should be low in order to have excellent mechanical transparency.

With rehabilitation robots, it should be generally ensured that the mechanism is retro-guidable.

The safety of the device is essential.

In [1] the problem of joint misalignment is discussed, i.e., the difficulty of obtaining the axes of the active robotic joints correctly aligned with those of the human body joints.

This phenomenon may cause unwanted arm movements and may simultaneously cause unwanted stresses on the human body, due to the hyperstatic properties of the human plus robot system. In fact, in the event of misalignment, if the two kinematic chains were both rigid, movement would be totally prevented; in reality the misalignment is recovered through deformations. The major deformations in the human plus robot system occur at the interface between human and robot given the low stiffness of skin, fat cells and muscle structure compared to the exoskeleton materials. That is why in the event of joint misalignment, unwanted stresses are put on the human exoskeleton interfaces.

In order to obviate to this problem, among the methods already used, it is possible to 1) insert elasticity and compliance within the exoskeleton, or 2) add redundant passive joints in the kinematic chain, or 3) use links of adjustable length or other alignment correction mechanisms, or still 4) use interfaces between exoskeleton and human with passive joints, in order to avoid unwanted stresses.

Due to the load transfer between robot and human as the interface surface area changes, it is clear that slightly extended interfaces can be uncomfortable if the interface transmits torque, therefore use of the widest possible interfaces or at most the use of pairs of interfaces is recommended.

For this reason, in recent years soft wearable robotic devices have been introduced, i.e. characterized by flexible links, or entirely made of light, flexible materials such as fabrics of textile fibers or typical fibers of composite materials. One of the first soft robotics works for assisting arms was obtained by Kobayashi and Hiramatsu, at the University of Tokyo [1].

This system substantially consists of a fabric suit, which is a sort of second skin for the torso and arms. McKibben-type pneumatic actuators are attached to this suit which function substantially like the muscles of the human body, i.e., they can contract to decrease the distance between two points of the suit.

Conor Walsh of Harvard University subsequently worked on soft wearable devices with pneumatic actuation. The basic concept is the same as the muscle suit of the University of Tokyo, that is, using air chambers as an external muscular system. As for the upper limb, Prof. Walsh substantially produced two devices, one for the hand and one for the shoulder [2-3].

Lorenzo Masia et al., at NTU University in Singapore, have developed an exosuit (soft wearable exoskeleton) for elbows [4, CN107921628] consisting of two fabric interfaces which are closable around the arm with Velcro straps. One of the interfaces is placed on the forearm and one on the arm. Two cables are interposed between these two interfaces, which act as the two antagonist muscles, the biceps and triceps. The cables are fixed between the two interfaces and guided by a motor positioned inside a rear backpack, with a Bowden cable transmission (sheathed cable).

At the state of the art there are numerous exoskeletal devices.

Most solutions provided in literature refer to solutions using rigid exoskeletal structures for the transmission of forces and movement.

In US2019160653 a parallel kinematic system is described for the actuation of the shoulder joint through a mechanism actuated with 3 linear actuators, while in WO2018093448 a kinematic mechanism with remote center of rotation and rigid-link spherical kinematics is described. Other similar devices can be found in documents WO 2017/216663 A1 and WO 2013/186705 A2.

Some of these systems are dedicated to supporting the lower limb and use pneumatic actuation (US2019029914) or systems of bands adapted to transfer tension up (CN108670195, US2018008502). The same concepts are applied through the use of Bowden cables in CN107921628, where a solution adapted to transfer an assistance torque to the elbow is provided.

In CN109318217A a Bowden cable actuation system is patented for the actuation of the flexion/extension or abduction/adduction joint of the shoulder joint, through the use of an actual pulley mounted on a rotoidal pair with vertical inclination. Such a system does not allow full movements of the shoulder joint.

In WO2019081851 an articulated, soft parallelogram mechanism is described which allows to position an axis actuated in alignment with a degree of freedom of the wearer's shoulder.

Instead, in ITUA20164364A1, a system for transferring a rotary motion between two arbitrary pulleys arranged on axes in space with arbitrary spatial orientation is provided.

Some mechanisms for transmission by shoulder exoskeleton are described in the following bibliographical references:
[1] H. Kobayashi, K. Hiramatsu, Development of muscle suit for upper limb. Proceedings of the IEEE International Conference on Robotics & Automation, 2004.
[2] P. Polygerinos, Z. Wang, K. C. Galloway, R. J. Wood, C. J. Walsh, Soft robotic glove for combined assistance and at-home rehabilitation. Robotics and Autonomous Systems, 2015, No. 73, 135-143
[3] C. T. O'Neill, N. S. Phipps, L. Cappello, S. Paganoni, C. J. Walsh, A soft wearable robot for the shoulder: design, characterization, and preliminary testing . International Conference on Rehabilitation Robotics, 2017
[4] Y. G. Kim, M. Xyloiannis, D. Accoto, L. Masia, Development of a soft exosuit for industrial application. 7th International Conference on Biomedical Robotics and Biomechatronics (BioRob), 2018.

The aforesaid known mechanisms for exoskeletons therefore do not allow to compensate for the movement of the shoulder center of rotation and recover misalignments.

### SUMMARY OF THE INVENTION

It is an object of the present invention to devise and provide an underactuated mechanism which allows to meet the aforesaid requirements and to at least partially overcome the drawbacks mentioned above with reference to the background art.

In particular, it is an object of the present invention to provide an underactuated mechanism for a robotic shoulder exoskeleton adapted to transfer the flexion-extension reaction force generated during the actuation of the shoulder joint through an actuator positioned at the level of the flexion extension shoulder joint in any configuration and orientation of the arm, through a flexible and kinematically hyper-redundant system capable of overcoming the abduction-adduction degrees of freedom of internal-external rotation and transferring the action on the torso of the person wearing the system, thus allowing the human to have greater freedom of movement on these degrees of freedom.

It is another object of the present invention to provide an underactuated mechanism for a robotic shoulder exoskeleton capable of bypassing the degrees of freedom of adduction-abduction and internal/external rotation of the shoulder and transferring the reaction required to balance the actuation torque at the level of the torso, thus allowing to implement an underactuated shoulder exoskeleton system.

It is another object of the present invention to provide an underactuated mechanism for a shoulder exoskeleton capable of correctly and automatically adapting the alignment of the axes of the active robotic joints with those of the human body joints.

These and further objects and advantages are achieved by an underactuated mechanism for shoulder exoskeleton, as well as by a shoulder exoskeleton comprising said underactuated mechanism, in accordance with the independent claims.

Further objects, solutions and advantages are found in the embodiments described below and claimed in the dependent claims.

In particular, the present underactuated mechanism allows, due to the kinematic structure thereof characterized by a proximal passive rotoidal joint and a distal active rotoidal joint, connected via a hyper-redundant mechanism, to create an exoskeleton for upper limbs which does not limit the human freedom of movement, because it has sufficient degrees of freedom for all the movements related to the shoulder joint, corresponding to a total of 3 independent degrees of freedom. The greater number of degrees of freedom of the aforesaid hyper-redundant mechanism connecting the two rotoidal joints also allows to have greater freedom of reconfiguration of the entire underactuated mechanism forming the exoskeleton, which therefore has the ability to self-align and compensate for the movements of the shoulder center of rotation and any assembly errors. Such an underactuated mechanism further has the peculiarity of always keeping the distal rotoidal joint always aligned with the flexion-extension axis of the shoulder, thus allowing to always assist the lifting of the arm with a single motor or actuation system. Finally, the particular kinematic structure of the aforesaid underactuated mechanism allows the reaction torque to be transmitted backwards, as there are no movement constraints between the rotation of the distal rotoidal joint and the hyper-redundant connection mechanism and the proximal rotoidal joint.

### BRIEF DESCRIPTION OF THE DRAWINGS

The invention will be illustrated below with the description of some embodiments thereof, given by way of non-limiting example, with reference to the accompanying drawings, in which:

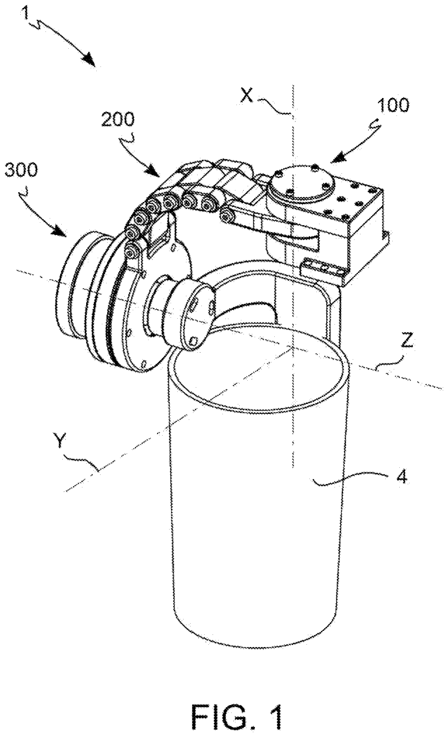

- figure 1 shows an isometric view of an underactuated mechanism according to the invention, which shows the three main modules which form the invention, i.e., the rotary mechanism which, once actuated, can transfer the flexion-extension torque action, the flexible element used for the torque transmission, the rotary mechanism integral with the person's torso;

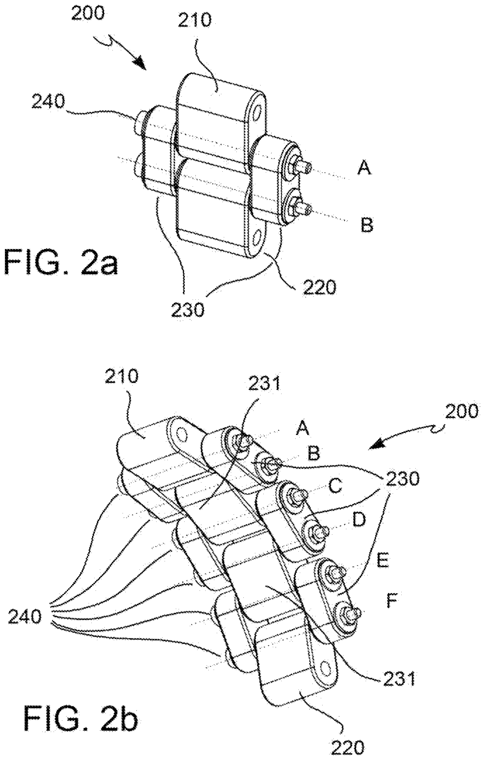

- figures 2a and 2b show a detail of the implementation of the transmission system, or hyper-redundant connection mechanism for transferring torque in the exemplary case of two members and four members, respectively;

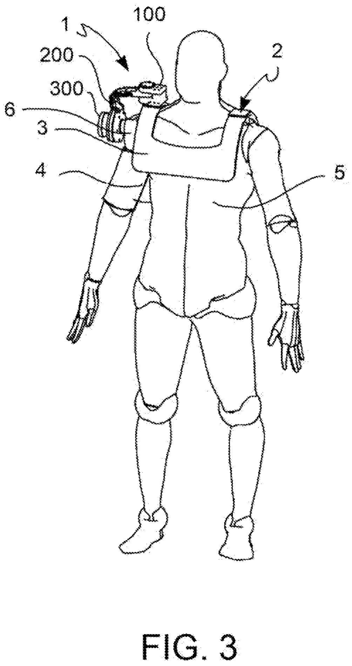

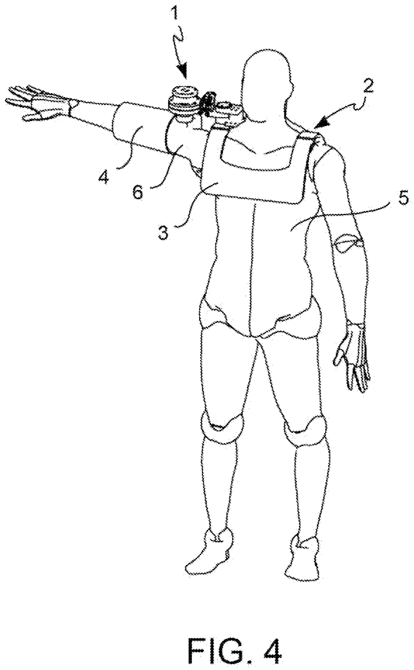

- figure 3 and figure 4 show two possible configurations of the shoulder exoskeleton comprising the underactuated mechanism of figure 1.

### DESCRIPTION OF THE PREFERRED EMBODIMENTS

With reference to the figures, an underactuated mechanism according to the invention, in particular for a robotic exoskeleton, is generally indicated with reference number 1.

The underactuated mechanism 1, which allows the actuation of a distal degree of freedom of the shoulder joint and the transfer of the torques and reaction force generated by the actuation system on a frame 3, or torso attachment device, integral with the torso, through a hyper-redundant kinematic mechanism 200, is formed as follows:
a. a first non-actuated rotoidal joint 100 connected, or adapted to be connected, to the human torso 5, which rotates about the axis X,
b. a connection mechanism 200 connected to the first rotoidal, or rotational, joint 100,
c. a second rotoidal, or rotational, joint 300 which (i) rotates about the axis Y arranged, or adapted to be arranged, according to the degree of shoulder joint flexion-extension, which is coplanar with the axis Y, (ii) is remotely actuated by a driven pulley and Bowden cables or by a direct drive actuation system with a co-located motor, (iii) is fastened to the connection mechanism 200 on one side and to the human arm 6 on the other side, with the connection mechanism 200 consisting of at least three or more members 210, 220, 231, two of which 210, 220 rigidly fastened to one of the rotation joints 100, 300, respectively, all with parallel joints, referred to as minimum joints A and B, arranged so as to connect one member with the next one to form a rotation constraint about the axis of said parallel joints.

In other words, the underactuated mechanism 1 comprises:
a. a first rotoidal joint 100, adapted to be connected to the human torso 5, said first rotoidal joint 100 defining a first joint rotation axis X;
b. a hyper-redundant connection mechanism 200 connected to the first rotoidal joint 100 at a first end of said hyper-redundant connection mechanism 200,
c. a second rotoidal joint 300 adapted to be connected to the human arm 6, said second rotoidal joint 300 defining a second joint rotation axis Y adapted to be arranged according to the degree of flexion-extension of the human shoulder joint, said second rotoidal joint 300 being connected to said hyper-redundant connection mechanism 200 at a second end of said hyper-redundant connection mechanism 200;

wherein said underactuated mechanism 1 is configured to transfer back torque and reaction forces from the second rotoidal joint 300 to the first rotoidal joint 100,
and wherein:
   - the first joint rotation axis X is coplanar with the second joint rotation axis Y;
   - the first rotoidal joint 100 is not actuated;
   - the second rotoidal joint 300 is actuated in a manner selected from: remotely by a driven pulley and Bowden cables, or by a direct drive actuation system with co-located motor;
   - the hyper-redundant connection mechanism 200 comprises at least three members 210, 220, 231 connected together by means of rotation joints with axes parallel to one another A, B, ... F, wherein a member 210, 220 of said at least three members is fastened to the first rotoidal joint 100 and another member of said at least three members 210 220 is fastened to the second rotoidal joint 300.

Thereby, the hyper-redundant connection mechanism 200 allows the passive rotation of the shoulder about the axis X perpendicular to the axes Y and Z, and capable of transmitting the constraining reaction torques perpendicular to the axis X and the constraining reaction forces along the axis X, without any relative rotation between the members forming the chain.

Furthermore, the connection mechanism is capable of varying the distance between the first rotoidal joint 100 and the second rotoidal joint 300, through a reconfiguration of the relative rotation between adjacent members.

According to one embodiment, only the second rotoidal joint 300 is actuated.

Direct drive actuation system with co-located motor means that the motor is integrated in the second rotoidal joint 300, or that the second rotoidal joint 300 is mounted directly on the output shaft of the motor.

In accordance with an embodiment, the underactuated mechanism 1 for shoulder exoskeleton lacks a forward kinematic transmission of rotation motion from said first rotoidal joint 100 to said second rotoidal joint 300.

That is, a rotation of the second rotoidal joint 300 about the second joint axis does not influence a rotation of the first rotoidal joint 100 about the first joint axis.

According to such an embodiment, the underactuated mechanism 1 transmits only a constraining reaction backwards from the second rotoidal joint 300 to the first rotoidal joint 100.

Within the scope of the present invention, it is assumed that an underactuated system is present, where the multi-degree of freedom mechanism has only the last degree of freedom actuated: moreover, a torque transfer between two axes is not required, since it is considered that the distal axis is provided with its own actuation through either a remote actuation system via Bowden or pneumatic cables, or on site (direct drive) by an actuator positioned on the joint.

"Underactuated" means a mechanism which has a lower number of degrees of freedom actuated with respect to the total number of degrees of freedom.

In other words, the mechanism 1 is underactuated since, between the first rotoidal joint 100 and the second rotoidal joint 300, one of the two is not actuated, in particular the first rotoidal joint is not actuated.

"Non-actuated rotoidal joint" means a system consisting of two bodies which can freely rotate with respect to each other along a certain axis, not provided with a rotary motor or other system capable of exerting an arbitrary value torque between the two parts, while "actuated rotoidal joint" means a system consisting of two bodies which can freely rotate with respect to each other along a certain axis, provided with a rotary motor or other actuation system capable of exerting an arbitrary value torque between the two sides.

"Hyper-redundant connection mechanism" means a serial mechanism with an overall number of degrees of freedom which is much greater than the number of degrees of freedom of the rigid end body of the mechanism.

"Serial mechanism" means a mechanism consisting of rigid bodies connected in series. Between two rigid bodies there is at least one joint allowing relative movement between the two bodies with at least one degree of freedom.

"Robotic shoulder exoskeleton" means a wearable mechanism, outside the human skeleton, which follows the kinematic structure thereof, and connected in one or more points to a human.

The first non-actuated rotoidal joint 100 is preferably arranged closer to the human torso than the second rotoidal joint 300.

For this reason, the first non-actuated rotoidal joint 100 can be defined proximal rotoidal joint, and the second actuated rotoidal joint 300 can be defined distal rotoidal joint.

Preferably, said one member of said at least three members 210 is arranged at a first end of said hyper-redundant connection mechanism 200, and in which said other of said at least three members 210 is arranged at a second opposite end of said hyper-redundant connection mechanism 200.

In accordance with an embodiment, the hyper-redundant connection mechanism 200 forms a chain of rigid bodies consisting of at least three different members 210, or rigid elements, connected together by means of coplanar rotoidal couplings along axes A,B,D...F... through rigid shafts 240 and spacers 230

In accordance with an embodiment, said at least three members 210 are rigid elements connected together in sequence, or together in series, to form a chain.

In accordance with an embodiment, each member of said at least three members 210 is connected to a subsequent member by means of two opposite connection spacer elements 230 of elongated shape and parallel to each other, each of said connection spacer elements 230 being rotatably engaged with said each member 210 and with said subsequent member about two of said parallel axes of said rotation joints, respectively.

In accordance with an embodiment, the underactuated mechanism 1 comprises elastic equilibrium means allowing the second rotoidal joint 300 to reach an equilibrium position with respect to the first rotoidal joint 100 determined by the minimum value of the elastic and gravitational potential.

For example, said elastic equilibrium means comprise elastic elements connected to at least two of said at least three members 210, or connected to said first rotoidal joint 100 and/or to said second rotoidal joint 300, and/or to at least one of said at least three members 210.

For example, said elastic equilibrium means comprise a metal foil or other elastic material, for example carbon fiber, which has a preferential elasticity in one plane and a high stiffness in the other two directions.

In other words, it is further possible to provide the system with an intrinsic elasticity through a system of elastic elements, which allows the chain to reach an equilibrium position determined by the minimum value of the elastic and gravitational potential.

In an alternative embodiment, the same constraint can be obtained through a metal foil or other material, for example carbon fiber, which has a preferential elasticity in one plane and a high stiffness in the other two directions.

In other words, the hyper-redundant connection device 200 can be provided with a distributed intrinsic elasticity system in order to achieve an equilibrium configuration, in the absence of external forces except for the weight thereof.

According to another aspect of the invention, the aforementioned objects and advantages are achieved by a robotic shoulder exoskeleton 2 comprising an underactuated mechanism 1 according to the features described above.

In particular, the exoskeleton can comprise a first torso attachment device 3 connected to said first non-actuated rotoidal joint 100, configured for fastening the underactuated mechanism 1 to the human torso 5, and an opposite second arm attachment device 4 connected to said second rotoidal joint 300, configured for fastening the underactuated mechanism 1 to the human arm 6.

The shoulder exoskeleton described above is capable of independently compensating for any misalignments between the human body joints and the exoskeleton joints by virtue of the hyper-redundant kinematic mechanism 200.

The shoulder exoskeleton described above, having the most distal rotational degree of freedom actuated, is capable of giving the wearer an assistance torque.

Although the underactuated mechanism 1 described above has been shown as applicable to a shoulder joint of the upper limb, it can similarly be applied to a knee joint of the lower limb, without substantial modifications.

Similarly, although a robotic shoulder exoskeleton of the upper limb has been described, such an exoskeleton can be a robotic knee exoskeleton of the lower limb, without substantial modifications.

Those skilled in the art may make modifications and adaptations to the embodiments of the device described above, or replace elements with others which are functionally equivalent, in order to meet contingent needs, without departing from the scope of the following claims. Each of the features described as belonging to a possible embodiment may be implemented irrespective of the other embodiments described.

## Claims

1. An underactuated mechanism (1), which allows the actuation of a distal degree of freedom of the human shoulder joint and the transfer of the reaction moments and force generated by the actuation system on the frame (3) solidal to the torso (5), through a hyper-redundant kinematic mechanism (200), composed of:
a. a first non-actuated rotoidal joint (100) connected to the human torso (5), which rotates about the axis X,
b. a connection mechanism (200) connected to the first rotoidal joint (100),
c. a second rotoidal joint (300) which (i) rotates about the axis Y arranged according to the degree of flexion-extension of the shoulder joint, which is coplanar with the axis X, (ii) is remotely actuated by a direct drive actuation system with co-located motor, (iii) is fixed to the connection mechanism (200) on one side and to the human arm (6) on the other side, with the connection mechanism (200) comprising: at least three or more members (210, 220, 231), two of which (210, 220) being rigidly fastened to one of the rotoidal joints (100, 300), respectively, all with parallel joints, referred to as minimum joints A and B, arranged so as to connect one member with the next one to form a rotation constraint about the axis of said parallel joints;
wherein the co-located motor is integrated in the second rotoidal joint (300).

2. An underactuated mechanism (1), according to claim 1, wherein only the second rotoidal joint (300) is actuated.

3. An underactuated mechanism (1) according to claim 1, wherein the underactuated mechanism (1) is devoid of a forward kinematic transmission of rotation motion from said first rotoidal joint (100) to said second rotoidal joint (300).

4. An underactuated mechanism (1) according to claim 3, wherein the underactuated mechanism (1) is configured to transmit only a constraining reaction backwards from the second rotoidal joint (300) to the first rotoidal joint (100).

5. An underactuated mechanism (1) according to claim 1, wherein the first non-actuated rotoidal joint (100) is arranged closer to the human torso (5) with respect to the second rotoidal joint (300).

6. An underactuated mechanism (1) according to claim 1, wherein said member of said at least three members (210) is arranged at a first end of said hyper-redundant connection mechanism (200), and wherein said other of said at least three members (210) is arranged at a second opposite end of said hyper-redundant connection mechanism (200).

7. An underactuated mechanism (1) according to claim 1, wherein the hyper-redundant connection mechanism (200) forms a chain of rigid bodies consisting of at least three different members (210), or rigid elements, connected together by means of coplanar rotoidal couplings along axes A,B,D...F... through rigid shafts (240) and spacers (230).

8. An underactuated mechanism (1) according to claim 1, wherein said at least three members (210) are rigid elements connected together in sequence, or together in series, to form a chain.

9. An underactuated mechanism (1) according to claim 1, wherein each member of said at least three members (210) is connected to a subsequent member by means of two opposite connection spacer elements (230) of elongated shape and parallel to each other, each of said connection spacer elements (230) being rotatably engaged with said each member (210) and said subsequent member about two of said parallel axes of said rotation joints, respectively.

10. An underactuated mechanism (1) according to claim 1, comprising elastic equilibrium means configured in order to hold the second rotoidal joint (300) in an equilibrium position with respect to the first rotoidal joint (100), said equilibrium position being determined by the minimum value of the elastic and gravitational potential of the second rotoidal joint (300) with respect to the first rotoidal joint (100).

11. An underactuated mechanism (1) according to claim 10, wherein said elastic equilibrium means comprise elastic elements connected to at least two of said at least three members (210), or connected to said first rotoidal joint (100) and/or to said second rotoidal joint (300), and/or to at least one of said at least three members (210).

12. An underactuated mechanism (1) according to claim 10, wherein said elastic equilibrium means comprise a metal foil or other elastic material, for example carbon fiber, which has a preferential elasticity in one plane and a high stiffness in the other two directions.

13. An underactuated mechanism (1) according to claim 1, wherein said second rotoidal joint (300) adapted to be connected to the human arm (6), said second rotoidal joint (300) defining a second joint rotation axis Y adapted to be arranged according to the degree of flexion-extension of the human shoulder joint, said second rotoidal joint (300) being connected to said hyper-redundant connection mechanism (200) at a second end of said hyper-redundant connection mechanism (200);
wherein said underactuated mechanism (1) is configured to transfer back torque and reaction forces from the second rotoidal joint (300) to the first rotoidal joint (100),
and wherein:
- the first joint rotation axis X is coplanar with the second joint rotation axis Y;
- the first rotoidal joint (100) is not actuated;
- the second rotoidal joint (300) is actuated in a manner selected from: remotely by a direct drive actuation system with co-located motor;
- the hyper-redundant connection mechanism (200) comprises at least three members (210, 220, 231) connected together by means of rotation joints with axes parallel to one another (A, B, ... F), wherein one member (210) of said at least three members is fastened to the first rotoidal joint (100) and another member (220) of said at least three members is fastened to the second rotoidal joint (300).

14. A robotic shoulder exoskeleton (2) comprising an underactuated mechanism (1) according to at least one preceding claim.

15. A robotic shoulder exoskeleton (2) according to claim 14, comprising a first torso attachment device (3), connected to said first non-actuated rotoidal joint (100), and configured for fastening the underactuated mechanism (1) to the human torso (5), and an opposite second arm attachment device (4) connected to said second rotoidal joint (300), and configured for fastening the underactuated mechanism (1) to the human arm (6).

## Patentansprüche

1. Unterbetätigter Mechanismus (1), welcher die Betätigung eines distalen Freiheitsgrads des menschlichen Schultergelenks und die Übertragung der Reaktionsmomente und der Kraft, welche durch das Betätigungssystem an dem Rahmen (3) erzeugt werden, solidal auf den Rumpf (5) überträgt, durch einen hyper-redundanten kinematischen Mechanismus (200), bestehend aus:
a. einem mit dem menschlichen Rumpf (5) verbundenen, ersten nicht betätigten rotoidalen Gelenk (100), welches sich um die Achse X dreht,
b. einem Verbindungsmechanismus (200), welcher mit dem ersten rotoidalen Gelenk (100) verbunden ist,
c. einem zweiten rotoidalen Gelenk (300), welches (i) sich um die Achse Y, welche gemäß dem Grad einer Beugung-Streckung des Schultergelenks angeordnet ist und welche koplanar mit der Achse X ist, (ii) durch ein Direktantriebsbetätigungssystem mit einem gemeinsam damit angeordneten Motor fernbetätigt wird, (iii) an dem Verbindungsmechanismus (200) an einer Seite und an dem menschlichen Arm (6) an der anderen Seite fixiert ist, wobei der Verbindungsmechanismus (200) umfasst: wenigstens drei oder mehr Elemente (210, 220, 231), von welchen zwei (210, 220) jeweils starr an einem der rotoidalen Gelenke (100, 300) befestigt sind, alle mit parallelen Gelenken, welche als Minimalgelenke A und B bezeichnet werden und welche derart angeordnet sind, dass sie ein Element mit dem nächsten verbinden, um eine Drehbeschränkung um die Achse der parallelen Gelenke zu bilden;
wobei der gemeinsam damit angeordnete Motor in dem zweiten rotoidalen Gelenk (300) integriert ist.

2. Unterbetätigter Mechanismus (1) nach Anspruch 1, wobei nur das zweite rotoidale Gelenk (300) betätigt wird.

3. Unterbetätigter Mechanismus (1) nach Anspruch 1, wobei der unterbetätigte Mechanismus (1) frei von einer kinematischen Vorwärtsübertragung einer Drehbewegung von dem ersten rotoidalen Gelenk (100) auf das zweite rotoidale Gelenk (300) ist.

4. Unterbetätigter Mechanismus (1) nach Anspruch 3, wobei der unterbetätigte Mechanismus (1) dazu eingerichtet ist, nur eine beschränkende Reaktion nach hinten von dem zweiten rotoidalen Gelenk (300) auf das erste rotoidale Gelenk (100) zu übertragen.

5. Unterbetätigter Mechanismus (1) nach Anspruch 1, wobei das erste nicht betätigte rotoidale Gelenk (100) in Bezug auf das zweite rotoidale Gelenk (300) näher an dem menschlichen Rumpf (5) angeordnet ist.

6. Unterbetätigter Mechanismus (1) nach Anspruch 1, wobei das Element der wenigstens drei Elemente (210) an einem ersten Ende des hyper-redundanten Verbindungsmechanismus (200) angeordnet ist und wobei das andere der wenigstens drei Elemente (210) an einem zweiten entgegengesetzten Ende des hyper-redundanten Verbindungsmechanismus (200) angeordnet ist.

7. Unterbetätigter Mechanismus (1) nach Anspruch 1, wobei der hyper-redundante Verbindungsmechanismus (200) eine Kette starrer Körper bildet, welche aus wenigstens drei Elementen (210), oder starren Elementen, besteht, die mittels koplanarer rotoidaler Kopplungen entlang Achsen A, B, D, ... F durch starre Wellen (240) und Abstandshalter (230) miteinander verbunden sind.

8. Unterbetätigter Mechanismus (1) nach Anspruch 1, wobei die wenigstens drei Elemente (210) starre Elemente sind, welche nacheinander, oder in Reihe, miteinander verbunden sind, um eine Kette zu bilden.

9. Unterbetätigter Mechanismus (1) nach Anspruch 1, wobei jedes Element der wenigstens drei Elemente (210) mittels zweier entgegengesetzter Verbindungsabstandshalterelementen (230) einer länglichen Form und parallel zueinander mit einem nachfolgenden Element verbunden ist, wobei jedes der Verbindungsabstandshalterelementen (230) mit dem jeden Element (210) bzw. dem nachfolgenden Element drehbar um zwei der parallelen Achsen der Drehgelenke in Eingriff gebracht ist.

10. Unterbetätigter Mechanismus (1) nach Anspruch 1, umfassend elastische Gleichgewichtsmittel, welche eingerichtet sind, um das zweite rotoidale Gelenk (300) in Bezug auf das erste rotoidale Gelenk (100) in einer Gleichgewichtsposition zu halten, wobei die Gleichgewichtsposition durch den minimalen Wert des Elastizitäts- und Gravitationspotentials des zweiten rotoidalen Gelenks (300) in Bezug auf das erste rotoidale Gelenk (100) bestimmt ist.

11. Unterbetätigter Mechanismus (1) nach Anspruch 10, wobei die elastischen Gleichgewichtsmittel elastische Elemente umfassen, welche mit wenigstens zwei der wenigstens drei Elemente (210) verbunden sind oder mit dem ersten rotoidalen Gelenk (100) und/oder mit dem zweiten rotoidalen Gelenk (300) und/oder mit wenigstens einem der wenigstens drei Elemente (210) verbunden sind.

12. Unterbetätigter Mechanismus (1) nach Anspruch 10, wobei die elastischen Gleichgewichtsmittel eine Metallfolie oder ein anderes elastisches Material, zum Beispiel Kohlenstofffaser, umfassen, welches eine bevorzugte Elastizität in einer Ebene und eine hohe Steifigkeit in den anderen beiden Richtungen aufweist.

13. Unterbetätigter Mechanismus (1) nach Anspruch 1, wobei das zweite rotoidale Gelenk (300) dazu eingerichtet ist, mit dem menschlichen Arm (6) verbunden zu sein, wobei das zweite rotoidale Gelenk (300) eine zweite Gelenkdrehachse Y definiert, welche dazu eingerichtet ist, gemäß dem Grad einer Beugung-Streckung des menschlichen Schultergelenks angeordnet zu sein, wobei das zweite rotoidale Gelenk (300) an einem zweiten Ende des hyper-redundanten Verbindungsmechanismus (200) mit dem hyper-redundanten Verbindungsmechanismus (200) verbunden ist;
wobei der unterbetätigte Mechanismus (1) dazu eingerichtet ist, ein Drehmoment und Reaktionskräfte von dem zweiten rotoidalen Gelenk (300) zurück auf das erste rotoidale Gelenk (100) zu übertragen,
und wobei:
- die erste Gelenkdrehachse X koplanar mit der zweiten Gelenkdrehachse Y ist;
- das erste rotoidale Gelenk (100) nicht betätigt wird;
- das zweite rotoidale Gelenk (300) in einer Weise betätigt wird, ausgewählt aus: entfernt durch ein Direktantriebsbetätigungssystem mit einem gemeinsam damit angeordneten Motor;
- der hyper-redundante Verbindungsmechanismus (200) wenigstens drei Elemente (210, 220, 231) umfasst, welche mittels Drehgelenken mit zueinander parallelen Achsen (A, B, ... F) miteinander verbunden sind, wobei ein Element (210) der wenigstens drei Elemente an dem ersten rotoidalen Gelenk (100) befestigt ist und ein anderes Element (220) der wenigstens drei Elemente an dem zweiten rotoidalen Gelenk (300) befestigt ist.

14. Roboter-Schulter-Exoskelett (2), umfassend einen unterbetätigten Mechanismus (1) nach wenigstens einem vorhergehenden Anspruch.

15. Roboter-Schulter-Exoskelett (2) nach Anspruch 14, umfassend eine erste Rumpfanbringungsvorrichtung (3), welche mit dem ersten nicht betätigten rotoidalen Gelenk (100) verbunden ist und dazu eingerichtet ist, den unterbetätigten Mechanismus (1) an dem menschlichen Rumpf (5) zu befestigen, und eine entgegengesetzte zweite Armanbringungsvorrichtung (4), welche mit dem zweiten rotoidalen Gelenk (300) verbunden ist und dazu eingerichtet ist, den unterbetätigten Mechanismus (1) an dem menschlichen Arm (6) zu befestigen.

## Revendications

1. Mécanisme sous-actionné (1), qui permet l'actionnement d'un degré de liberté distal de l'articulation d'épaule humaine et le transfert des moments et force de réaction générés par le système d'actionnement sur l'armature (3) solidaire du torse (5), par l'intermédiaire d'un mécanisme cinématique hyper-redondant (200), composé de :
a. une première articulation rotoïde non actionnée (100) raccordée au torse humain (5), qui tourne autour de l'axe X,
b. un mécanisme de raccordement (200) raccordé à la première articulation rotoïde (100),
c. une deuxième articulation rotoïde (300) qui (i) tourne autour de l'axe Y agencé selon le degré de flexion-extension de l'articulation d'épaule, qui est coplanaire avec l'axe X, (ii) est actionnée à distance par un système d'actionnement à entraînement direct avec moteur colocalisé, (iii) est fixée au mécanisme de raccordement (200) sur un côté et au bras humain (6) sur l'autre côté, le mécanisme de raccordement (200) comprenant : au moins trois organes ou plus (210, 220, 231), dont deux (210, 220) sont fixés rigidement à l'une des articulations rotoïdes (100, 300), respectivement, tous avec des articulations parallèles, appelées articulations minimales A et B, agencées de manière à raccorder un organe au suivant pour former une contrainte de rotation autour de l'axe desdites articulations parallèles ;
dans lequel le moteur colocalisé est intégré dans la deuxième articulation rotoïde (300).

2. Mécanisme sous-actionné (1) selon la revendication 1, dans lequel seule la deuxième articulation rotoïde (300) est actionnée.

3. Mécanisme sous-actionné (1) selon la revendication 1, dans lequel le mécanisme sous-actionné (1) est dépourvu de transmission cinématique vers l'avant du mouvement de rotation de ladite première articulation rotoïde (100) à ladite deuxième articulation rotoïde (300).

4. Mécanisme sous-actionné (1) selon la revendication 3, dans lequel le mécanisme sous-actionné (1) est configuré pour transmettre uniquement une réaction de contrainte vers l'arrière de la deuxième articulation rotoïde (300) à la première articulation rotoïde (100).

5. Mécanisme sous-actionné (1) selon la revendication 1, dans lequel la première articulation rotoïde non actionnée (100) est disposée plus proche du torse humain (5) par rapport à la deuxième articulation rotoïde (300).

6. Mécanisme sous-actionné (1) selon la revendication 1, dans lequel ledit organe desdits au moins trois organes (210) est disposé à une première extrémité dudit mécanisme de raccordement hyper-redondant (200), et dans lequel ledit autre desdits au moins trois organes (210) est disposé à une deuxième extrémité opposée dudit mécanisme de raccordement hyper-redondant (200).

7. Mécanisme sous-actionné (1) selon la revendication 1, dans lequel le mécanisme de raccordement hyper-redondant (200) forme une chaîne de corps rigides constituée d'au moins trois organes différents (210), ou organes rigides, raccordés ensemble au moyen de couplages rotoïdes coplanaires le long d'axes A, B, D... F... par l'intermédiaire d'arbres rigides (240) et de dispositifs d'espacement (230).

8. Mécanisme sous-actionné (1) selon la revendication 1, dans lequel lesdits au moins trois organes (210) sont des éléments rigides raccordés ensemble en séquence, ou ensemble en série, pour former une chaîne.

9. Mécanisme sous-actionné (1) selon la revendication 1, dans lequel chaque organe desdits au moins trois organes (210) est raccordé à un organe suivant au moyen de deux éléments d'espacement de raccordement opposés (230) de forme allongée et parallèles l'un à l'autre, chacun desdits éléments d'espacement de raccordement (230) étant en prise de manière rotative avec chaque organe (210) et ledit organe suivant autour de deux desdits axes parallèles desdites articulations de rotation, respectivement.

10. Mécanisme sous-actionné (1) selon la revendication 1, comprenant des moyens d'équilibre élastique configurés pour maintenir la deuxième articulation rotoïde (300) dans une position d'équilibre par rapport à la première articulation rotoïde (100), ladite position d'équilibre étant déterminée par la valeur minimale du potentiel élastique et gravitationnel de la deuxième articulation rotoïde (300) par rapport à la première articulation rotoïde (100).

11. Mécanisme sous-actionné (1) selon la revendication 10, dans lequel lesdits moyens d'équilibre élastique comprennent des éléments élastiques raccordés à au moins deux desdits au moins trois organes (210), ou raccordés à ladite première articulation rotoïde (100) et/ou à ladite deuxième articulation rotoïde (300), et/ou à au moins l'un desdits au moins trois organes (210).

12. Mécanisme sous-actionné (1) selon la revendication 10, dans lequel lesdits moyens d'équilibre élastique comprennent une feuille métallique ou un autre matériau élastique, par exemple de la fibre de carbone, qui a une élasticité préférentielle dans un plan et une rigidité élevée dans les deux autres directions.

13. Mécanisme sous-actionné (1) selon la revendication 1, dans lequel ladite deuxième articulation rotoïde (300) est adaptée pour être raccordée au bras humain (6), ladite deuxième articulation rotoïde (300) définissant un deuxième axe de rotation d'articulation Y adapté pour être agencé en fonction du degré de flexion-extension de l'articulation d'épaule humaine, ladite deuxième articulation rotoïde (300) étant raccordée audit mécanisme de raccordement hyper-redondant (200) à une deuxième extrémité dudit mécanisme de raccordement hyper-redondant (200) ;
dans lequel ledit mécanisme sous-actionné (1) est configuré pour retransférer le couple et les forces de réaction de la deuxième articulation rotoïde (300) à la première articulation rotoïde (100),
et dans lequel :
- le premier axe de rotation d'articulation X est coplanaire avec le deuxième axe de rotation d'articulation Y ;
- la première articulation rotoïde (100) n'est pas actionnée ;
- la deuxième articulation rotoïde (300) est actionnée d'une manière choisie parmi : à distance par un système d'actionnement à entraînement direct avec moteur colocalisé ;
- le mécanisme de raccordement hyper-redondant (200) comprend au moins trois organes (210, 220, 231) raccordés ensemble au moyen d'articulations de rotation ayant des axes parallèles entre eux (A, B,... F), dans lequel un organe (210) desdits au moins trois organes est fixé à la première articulation rotoïde (100) et un autre organe (220) desdits au moins trois organes est fixé à la deuxième articulation rotoïde (300).

14. Exosquelette d'épaule robotisé (2) comprenant un mécanisme sous-actionné (1) selon au moins une revendication précédente.

15. Exosquelette d'épaule robotisé (2) selon la revendication 14, comprenant un premier dispositif d'attache au torse (3), raccordé à ladite première articulation rotoïde non actionnée (100), et configuré pour fixer le mécanisme sous-actionné (1) au torse humain (5), et un deuxième dispositif d'attache au bras opposé (4) raccordé à ladite deuxième articulation rotoïde (300), et configuré pour fixer le mécanisme sous-actionné (1) au bras humain (6).
